# EUROPEAN PATENT APPLICATION

(11) **EP 3 586 657 A2**
(43) Date of publication of application: **01.01.2020**
(21) Application number: 19179301.7
(22) Date of filing: 11.06.2019
(51) Int. Cl.: A24F 47/00

(54) **E-LIQUID DELIVERY SYSTEM FOR PERSONAL VAPORIZERS**

(30) Priority: 21.06.2018 US 201862687883 P
(71) Applicant: Avail Vapor, LLC, Richmond, Virginia 23236 (US)
(72) Inventor: ANGELICO, Vincent, Richmond, VA Virginia 23236 (US); ROGERS, Russell, Richmond, VA Virginia 23236 (US); ATHAYDE, Matt, Richmond, VA Virginia 23236 (US)
(74) Representative: Latham, Stuart Alexander

(57) **Abstract**

A personal vaporizer has a vaporization chamber within a main body interior, a vaporization heating element disposed within the vaporization chamber, an air flow passage from one or more air intake openings in the case wall to the vaporization chamber, and a vaporization mixture flow passage from the vaporization chamber to an exit port. The personal vaporizer also has a liquid reservoir and a liquid transport arrangement for transporting vaporizable liquid from the liquid reservoir to the vaporization chamber. The liquid transport arrangement has a liquid flow duct extending from the reservoir exit port and a piezoelectric pump. The piezoelectric pump is operatively connected to the liquid flow duct for drawing the vaporizable liquid from the reservoir, through the liquid flow duct, and into the vaporization chamber through a nozzle so that liquid exiting the nozzle is heated by the vaporization heating surface.

## Description

This application claims priority to U.S. Provisional Patent Application 62/687,883, filed June 21, 2018, the complete disclosure of which is incorporated herein by reference.

### BACKGROUND OF THE INVENTION

The invention relates generally to micro-vaporizers and, more particularly, to a wickless liquid delivery system for personal micro-vaporizers.

Micro-vaporizers are devices in which a vaporizable liquid (sometimes referred to as "e-liquid") is drawn from a storage reservoir into a chamber where it is heated to vaporization temperature by a heating element. The vaporized liquid is then drawn or forced from the chamber. In products such as electronic cigarettes (also known as e-cigarettes or personal vaporizers), the vaporized liquid is drawn from the chamber through a mouthpiece and inhaled by the user. In other products the vaporized liquid is dispersed into the atmosphere.

The usual purpose of a device that uses a micro-vaporizer is to dispense one or more active substances using the vaporized liquid. In atmospheric dispensers, these substances may include materials such as deodorizing agents, fragrance, and insect repellant. In the case of personal vaporizers, the active substances typically include a flavorant (i.e., a flavoring agent or material) and nicotine. The flavorant and nicotine levels may be selected so as to mimic the experience of smoking a cigarette.

### SUMMARY OF THE INVENTION

An illustrative aspect of the invention provides a personal vaporizer comprising an annular main body defining a main body interior, a vaporization chamber within the main body interior, and a vaporization heating element disposed within the vaporization chamber. The vaporization heating element has a vaporization heating surface. The personal vaporizer further comprises an air flow passage from one or more air intake openings in the case wall to the vaporization chamber and a vaporization mixture flow passage extending from the vaporization chamber to an exit port. The personal vaporizer still further comprises a liquid reservoir and a liquid transport arrangement for transporting vaporizable liquid from the liquid reservoir to the vaporization chamber. The liquid reservoir is configured for storage of a vaporizable liquid and has a reservoir exit port. The liquid transport arrangement comprises a first liquid flow duct extending from the reservoir exit port and a piezoelectric pump. The piezoelectric pump is operatively connected to the first liquid flow duct for drawing the vaporizable liquid from the reservoir, through the liquid flow duct, and into the vaporization chamber through a nozzle positioned adjacent or in contact with the vaporization heating element so that liquid exiting the nozzle is heated by the vaporization heating surface.

Another illustrative aspect of the invention provides a method of vaporizing a vaporizable liquid in a personal vaporizer having a liquid reservoir containing the vaporizable liquid, a vaporization chamber, and a vaporization heating element disposed within the vaporization chamber. The method comprises drawing the vaporizable liquid from the liquid reservoir using an internal pumping mechanism and pre-heating, by a first heating element, the vaporizable liquid to a temperature within a predetermined temperature range. The pre-heated vaporizable liquid is passed through a nozzle into the vaporization chamber adjacent the vaporization heating element. The method further comprises heating, by a second heating element, the vaporizable liquid above a vaporization temperature, thereby producing a vapor product.

### BRIEF DESCRIPTION OF THE DRAWINGS

The invention can be more fully understood by reading the following detailed description together with the accompanying drawing, in which like reference indicators are used to designate like elements, and in which:
Figure 1 is a cross-sectional view of a prior art personal vaporizer;
Figure 2 is a cross-sectional view of a personal vaporizer according to an embodiment of the invention;
Figure 3 is a block diagram representation of a vaporizer system incorporating liquid preheating according to an embodiment of the invention;
Figure 4 is a cross-sectional view of a personal vaporizer according to an embodiment of the invention; and
Figure 5 is a cross-sectional view of a fluid transport arrangement according to an embodiment of the invention.

### DETAILED DESCRIPTION OF THE INVENTION

The invention will be described using examples and embodiments geared primarily to personal vaporizers. It will be understood, however, that the methods of the invention are not limited to such applications and can be applied to any micro-vaporizer device.

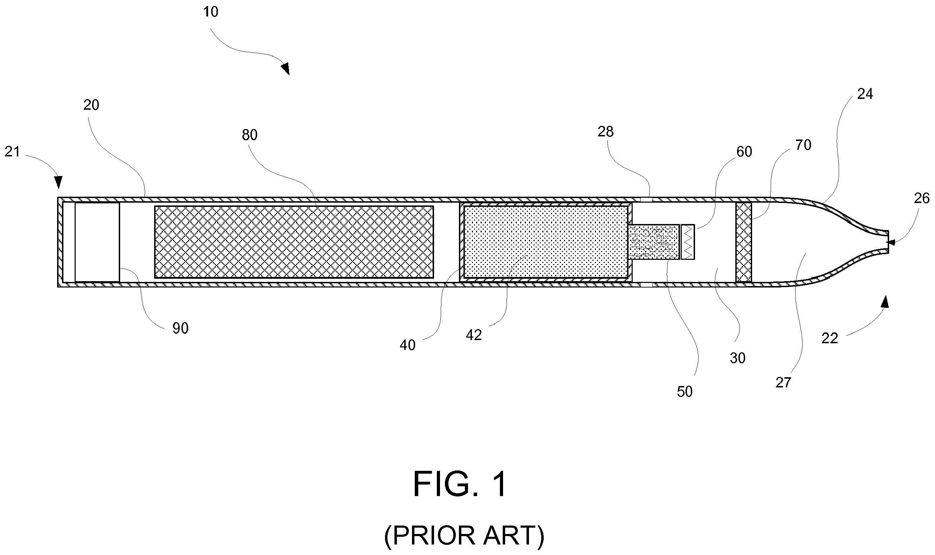

With reference to Figure 1, a typical personal vaporizer 10 comprises a cylindrical casing 20 having a distal end 21 and a proximal end 22. At its proximal end 22, the casing 20 is formed into a mouthpiece 24 having a passage 26 providing fluid communication between the atmosphere and an exit chamber 27 inside the casing 20. The casing 20 also has one or more air holes 28 to allow air to flow from the atmosphere into a vaporization chamber 30 inside the casing 20 when a relative vacuum is applied at the mouthpiece passage 26 (e.g., by inhalation of a device user). The air drawn in through the air hole(s) 28 passes through a filter 70 which divides the vaporization chamber 30 and the exit chamber 27.

The personal vaporizer 10 further comprises a fluid reservoir 40 in which is disposed a vaporizable liquid 42. The fluid reservoir 40 may be configured as a simple tank in which the liquid 42 is disposed. In some embodiments, the reservoir 40 may be or include a housed or unhoused adsorptive or absorptive material or structure that retains the vaporizable liquid 42. A fluid transport structure 50 is configured and positioned to be in contact with the liquid 42 in the reservoir 40 and for drawing the liquid 42 out of the reservoir 40 and into the vaporization chamber 30. The fluid transport structure 50 may be further configured for bringing the drawn liquid 42 into close proximity or in contact with a heating element 60. The heating element 60 may be configured to heat the vaporizable liquid through any conductive, convective, and/or radiative heat transfer mechanism. In typical vaporizers, the heating element 60 is or includes a resistance element in the form of a wire coil. In some cases, the resistance element is housed within a heat conductive casing.

The illustrative personal vaporizer 10 also comprises a battery 80 for powering the heating element and a control unit 90. It will be understood that the configuration and relative positioning of the components of the personal vaporizer 10 may be widely varying and that additional components (e.g., an airflow controller for regulation of the amount of air flow through the holes 28) may be included.

The vaporizable liquid 42 typically includes an active material or substance. As used herein, the term "active material" refers to any material that controllably alters or adds to the vaporization products of the device. Depending on the application, active materials can include, without limitation, plant material, minerals, deodorizing agents, fragrances, insect repellants, medications, and disinfectants and any material or structure containing or incorporating any of the foregoing. In the specific instance of personal vaporizers, active materials may include flavorant substances that augment the flavor of the vaporizable liquid.

To use the personal vaporizer 10, a user activates the heating element 60 and draws air through the device by inhaling through the mouthpiece. The vaporizable liquid 42 in the chamber 30 is heated to its vaporization point by the heating element 60. The resulting vapor mixes with air drawn through the air holes 28 and the mixture is drawn through the filter 70 and the exit chamber 27 and out through the mouthpiece passage 26.

The fluid transport structure 50 of the personal vaporizer 10 typically comprises a wick or collection of wicking material that serves to draw liquid 42 from the reservoir 40. Flow potential through the wick is maintained by evaporation of liquid at the downstream end of the wick as it is heated by the heating element 60.

Use of a wicking material as a fluid transport mechanism has inherent limitations. Of particular interest in relation to the present invention is the inability to actively control the flow rate of the liquid and, thus, the rate at which liquid is delivered to and vaporized within the vaporization chamber. The use of wicks also leads to leakage issues, in part because the flow path from the reservoir is always open.

Attempts have been made to replace wicks with mechanical pumps and/or pressurized delivery mechanisms, but such mechanisms have their own limitations and are may be too bulky or heavy for use in personal vaporizers.

An aspect of the present invention solves the problem by using a piezoelectric (PZE) pump as a fluid transport mechanism. Piezoelectric pumps are compact diaphragm pumps that that can provide continuous or variable flow rates. They have been used in various applications requiring reliable metering of liquids and gases, including, for example, ink delivery systems in ink-jet printers.

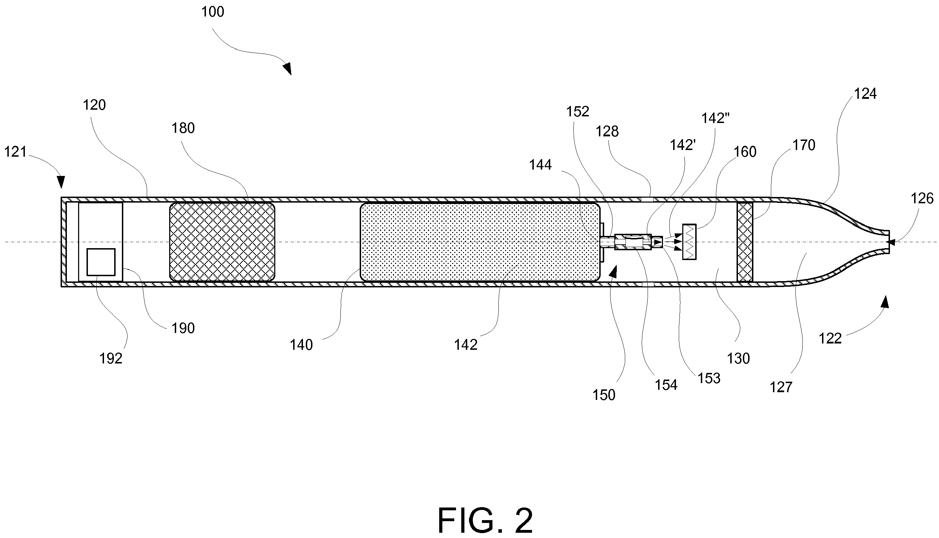

With reference to Figure 2, a personal vaporizer 100 according to a representative embodiment of the invention is similar in most respects to the personal vaporizer 10 of Figure 1. The vaporizer 100 comprises a cylindrical casing 120 having a distal end 121 and a proximal end 122. At its proximal end 122, the casing 120 is formed into a mouthpiece 124 having a passage 126. The casing 120 also has one or more air holes 128 to allow air to flow from the atmosphere into a vaporization chamber 130 which may be separated from the exit chamber 127 by a filter 170. The personal vaporizer 100 further comprises a fluid reservoir 140 in which is disposed a vaporizable liquid 142. The fluid reservoir 140 may be configured as a simple tank or may be or include a housed or unhoused adsorptive or absorptive material or structure that retains the vaporizable liquid 142. In either case, the reservoir 140 has a proximal reservoir exit port 144. A heating element 160 is positioned within the vaporization chamber 130. The personal vaporizer 100 also comprises a battery 180 and a control unit 190.

The personal vaporizer 100 differs from the vaporizer 10 in that it has a fluid transport structure 150 that comprises a conduit 152 extending proximally from the reservoir exit port 144 and a PZE pump 154. The conduit 152 and the PZE pump 154 are configured for drawing liquid 142 from the reservoir 140 and controlling flow of the liquid 142' through the conduit 152. The conduit 152 terminates in a nozzle end 153 that is positioned in close proximity or in contact with the heating element 160, which is configured to heat and vaporize the liquid 142" exiting the nozzle end 153. As before, the heating element 160 may be configured to heat the vaporizable liquid 142" through any conductive, convective, and/or radiative heat transfer mechanism. The nozzle end 153 may be configured to direct the liquid 142" in a particular disbursement pattern to enhance vaporization performance.

Operation of the PZE pump 154 may be controlled through the use of a controller 192, which may be part of the control unit 190. The PZE pump 154 and the controller 192 may be configured to control the rate of flow of the liquid 142' and, thus, the amount of liquid 142" delivered to the vaporization chamber 130. The PZE pump 154 may also be configured to selectively close off the conduit 152 to prevent unwanted flow of liquid 142 from the reservoir 140. In some embodiments, the control unit 190 may be in communication with a user input device (not shown) that allows the user to enter or select a desired liquid flow rate.

It will be understood that the configuration and relative positioning of the components of the personal vaporizer 100 may be widely varying. In some embodiments, for example, the conduit 152 may comprise one or more flexible tubes, the use of which would allow variable positioning of the exit port 144.

Use of the personal vaporizer 100 is substantially similar to that of the prior art vaporizer 10 except that activation of the heating element 160 also activates the pump 154, causing liquid 142 to be drawn from the reservoir 142 into the vaporization chamber 130 where it is heated to its vaporization point by the heating element 160. The resulting vapor mixes with air drawn through the air holes 128 and the mixture is drawn through the filter 170 and the exit chamber 127 and out through the mouthpiece passage 126. In some embodiments, an external switch or other user input device (not shown) in communication with the pump controller 192 may be manipulated by the user to change the flow rate of the liquid 142, which effectively changes the amount of liquid 142" vaporized and the rate of delivery of active materials in the inhaled vaporization products.

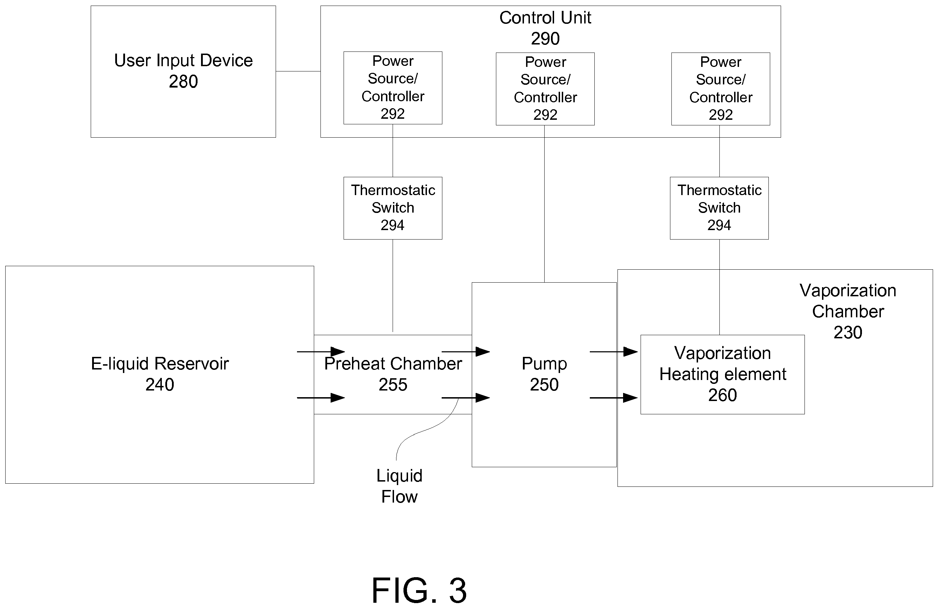

It has been found that the ability to controllably pump vaporizable liquids can be significantly affected by their viscosities. In some applications, it may be desirable to reduce the viscosity of a vaporizable liquid to enhance its flow properties and to achieve a precise, repeatable rate of delivery to the vaporization chamber. In many instances, this can be achieved by preheating the vaporizable liquid upstream of the micro-pump. Figure 3 is a block diagram of a generalized vaporizer system 200 with a pre-heating arrangement. As shown in the block diagram, a micro-pump 250 such as a PZE pump is used to draw liquid from a reservoir 240 and into contact with a vaporization heating element 260 in a vaporization chamber 230. Prior to the liquid passing through the pump 250, however, the liquid passes through a preheat chamber 255, where the liquid undergoes temperature conditioning. Through preheating, the liquid is brought to a temperature selected to produce a desired viscosity, which, in turn, is tailored to allow precise, metered flow control of the liquid through the pump 250 and into the vaporization chamber 230. In some embodiments and for typical e-liquids, the desired temperature may be in a range of 75-95 °F. In particular embodiments, the desired temperature may be in a range of 78-82 °F and is preferably about 80 °F. The preheat chamber 255, pump 250, and primary heating element 260 are all controllable through control modules 292 of a main control unit 290 of the device. Temperature control for the preheat chamber 255 and heating element 260 may be accomplished through the use of thermostatic switches 294.

The system 200 may optionally include a user input arrangement or device 280 in electrical communication with the control unit 290. The user input arrangement 280 may be configured for receiving inputs from the device user in relation to operation of the vaporizer system 200. In particular, the user input arrangement 280 may be arranged to receive a desired liquid flow rate, which may be transmitted to the control module 292 for the pump 250. The user input arrangement 280 could be or include any mechanism for receiving input. In some embodiments, the input arrangement may include a wireless communication module configured to receive user commands from a separate wireless communication device (e.g., a mobile phone, smart pad, or computer).

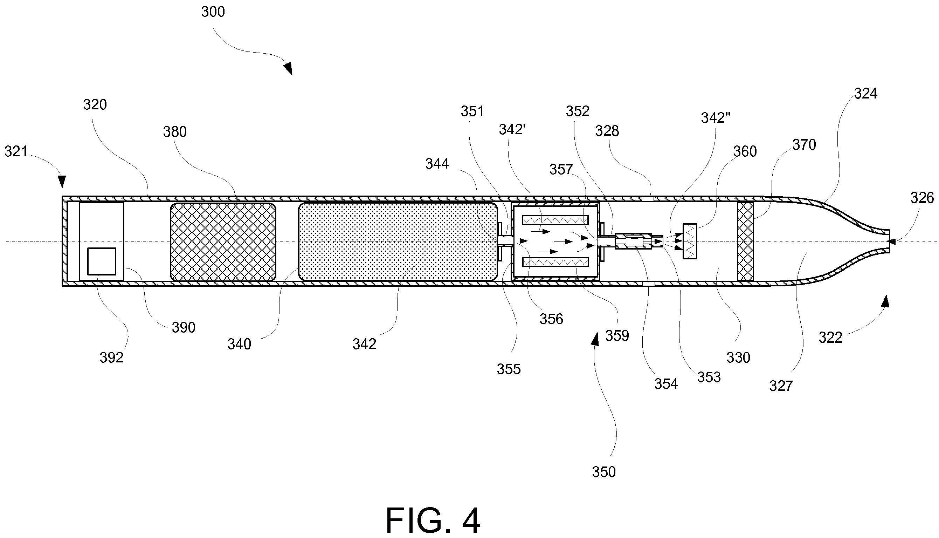

There are many possible configurations of personal vaporizers that could be used to implement the preheating methodology of the invention. Figure 4 illustrates an illustrative embodiment of a personal vaporizer 300 that incorporates this methodology. The vaporizer 300 comprises a cylindrical casing 320 having a distal end 321 and a proximal end 322. At its proximal end 322, the casing 320 is formed into a mouthpiece 324 having a passage 326. The casing 320 also has one or more air holes 328 to allow air to flow from the atmosphere into a vaporization chamber 330 which may be separated from the exit chamber 327 by a filter 370. The personal vaporizer 300 further comprises a fluid reservoir 340 in which is disposed a vaporizable liquid 342. The fluid reservoir 340 may be configured as a simple tank or may be or include a housed or unhoused adsorptive or absorptive material or structure that retains the vaporizable liquid 342. In either case, the reservoir 340 has a proximal reservoir exit port 344. A heating element 360 is positioned within the vaporization chamber 330. The personal vaporizer 300 also comprises a battery 380 and a control unit 390.

The personal vaporizer 300 has a fluid transport arrangement 350 that includes a preheat chamber 355 fluidly connected to the reservoir 340 by a first fluid conduit 351. The preheat chamber 355 is configured to allow flow of liquid 342' from an entrance port 356, through the chamber 355 and out through an exit port 357 on its downstream (proximal) side. The fluid transport structure 350 further includes a second conduit 352 and a PZE pump 354. The conduit 352 extends proximally from the exit port 357 of the preheat chamber 355. The conduit 352 terminates in a nozzle end 353 that is positioned in close proximity or in contact with the heating element 360, which is configured to heat and vaporize the liquid 342" exiting the nozzle end 353. The PZE pump 354 is configured for drawing liquid 342 from the reservoir 340 and through the preheat chamber 355 and for controlling flow of the liquid 342' through the conduit 352 for delivery into the vaporization chamber 330. The PZE pump 354 may be controlled by a pump controller 392 within the control unit 390.

Within the preheat chamber is/are disposed one or more preheating elements 359. These preheating elements 359 may be similar to the primary heating element 360, but with lower heating rates designed to raise the temperature of the liquid to a desired temperature to establish the desired flow properties upstream of the PZE pump 354. The preheating elements 359 may be configured for direct contact with or full immersion in the vaporizable liquid 342' flowing through the preheat chamber.

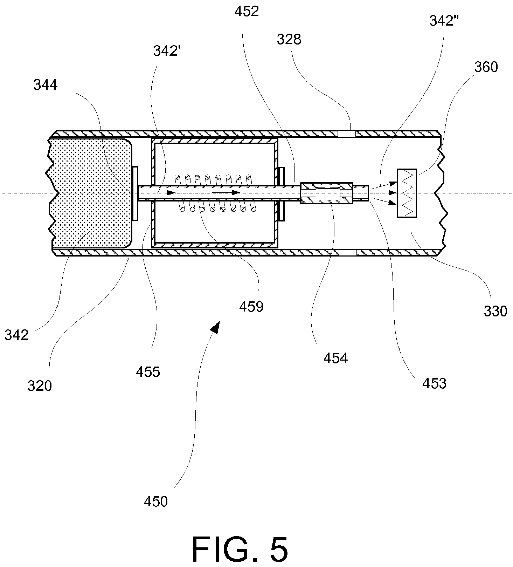

Figure 5 illustrates an alternative fluid transport arrangement 450 that could be used in the personal vaporizer 300. In this embodiment, the fluid transport arrangement 450 has a single fluid transport conduit 452 extending from the reservoir exit 344 and through the preheat chamber 455 to the PZE pump 454. Within the preheat chamber 455, a coiled heating element 459 is positioned so as to surround the conduit 452. The heating element 459 may be separated from, in contact with, or incorporated into the conduit 452 and is configured to heat liquid 342' passing through the conduit 452 so as to establish desired flow properties upstream of the PZE pump. The PZE pump 453 and nozzle end 453 are substantially similar to those of the previous embodiments.

Although the conduit 452 is illustrated as a straight tube passing through the preheat chamber 455, it will be understood that it may be configured with bends to increase its length and, thus, the dwell time of liquid 342' within the preheat chamber 455.

In both of the above embodiments, the heating elements 359, 459 may be controlled through controller elements within the control unit 390 of the personal vaporizer 300. In all of the various embodiments of the invention, control of the various heating elements may incorporate temperature sensors and/or thermostatic control elements. In the case of heating elements used to preheat the vaporizable liquid, a thermostatic switch may be used to assist in reproducibly achieving flow viscosities within desired limits and avoid excessive thinning of the liquid. Thermostatic switches can be used in primary heating elements as well to avoid overheating of the liquid, which can cause the formation of undesirable compounds.

In all of the embodiments disclosed herein, including the prior art vaporizer 10 of Figure 1, the various heating elements-particularly those that may be come in direct contact with the vaporizable liquid-may be replaced with elements formed from graphite/carbon fibers. The advantage of elements formed from such fibers is that they will not react or decompose at the high temperatures needed to vaporize the vaporizable liquids used in the vaporizers of the invention. This is particularly significant in personal vaporizers, because it presents little or no chance of contributing undesirable constituents (e.g., heavy metals) to the vaporization products of the device, either through interfacial thermal reaction or chemical leaching to the e-liquid or the vapor.

While the foregoing illustrates and describes exemplary embodiments of this invention, it is to be understood that the invention is not limited to the construction disclosed herein. The invention can be embodied in other specific forms without departing from the spirit or essential attributes.
Before setting out the claims, we will first present a set of clauses defining embodiments of the invention.
Clause 1. A personal vaporizer comprising:
   an annular main body defining a main body interior;
   a vaporization chamber within the main body interior;
   a vaporization heating element disposed within the vaporization chamber, the vaporization heating element having a vaporization heating surface;
   an air flow passage from one or more air intake openings in the case wall to the vaporization chamber;
   a vaporization mixture flow passage extending from the vaporization chamber to an exit port;
   a liquid reservoir configured for storage of a vaporizable liquid and having a reservoir exit port;
   a liquid transport arrangement for transporting vaporizable liquid from the liquid reservoir to the vaporization chamber, the liquid transport arrangement comprising
      a first liquid flow duct extending from the reservoir exit port, and
      a piezoelectric pump operatively connected to the first liquid flow duct for drawing the vaporizable liquid from the reservoir, through the liquid flow duct, and into the vaporization chamber through a nozzle positioned adjacent or in contact with the vaporization heating element so that liquid exiting the nozzle is heated by the vaporization heating surface.
Clause 2. A personal vaporizer according to claim 1 further comprising:
   a pump controller in electrical communication with the piezoelectric pump and configured for controlling the piezoelectric pump so as to maintain a desired flow rate of liquid through the nozzle.
Clause 3. A personal vaporizer according to claim 2 wherein the pump controller is configured to control the liquid flow rate as a function of a viscosity of the vaporizable liquid.
Clause 4. A personal vaporizer according to claim 2 further comprising:
   a user input device in electrical communication with the pump controller, the user input arrangement being configured for receiving a flow control input from a user of the personal vaporizer and transmitting the flow control input to the pump controller.
Clause 5. A personal vaporizer according to claim 4 further wherein the flow control input is the desired flow rate.
Clause 6. A personal vaporizer according to claim 4 wherein the flow control input is a binary on-off input and the pump controller is configured to command the piezoelectric pump to cause liquid to flow through the nozzle at the desired flow rate in response to an on command and to prevent liquid flow through the nozzle in response to an off command.
Clause 7. A personal vaporizer according to claim 2 wherein the desired flow rate is in a range from zero flow to a predetermined maximum flow rate.
Clause 8. A personal vaporizer according to claim 1 wherein the liquid transport arrangement further comprises:
   a pre-heat chamber having a pre-heat chamber inlet in fluid communication with the first liquid flow duct and a pre-heat chamber outlet;
   a second liquid flow duct in fluid communication with the pre-heat chamber outlet and the piezoelectric pump for transporting the vaporizable liquid therebetween; and
   a liquid preheat heating element disposed within the pre-heat chamber.
Clause 9. A personal vaporizer according to claim 8 wherein the pre-heat chamber and the liquid preheat heating element are configured to heat the vaporizable liquid to a liquid temperature in a range of 75-90 °F.
Clause 10. A personal vaporizer according to claim 8 wherein the pre-heat chamber and the liquid preheat heating element are configured to heat the vaporizable liquid to a liquid temperature in a range of 78-82 °F.
Clause 11. A personal vaporizer according to claim 1 wherein the liquid transport arrangement further comprises:
   a pre-heat chamber within which at least a portion of the first liquid flow duct is disposed; and
   at least one liquid preheat heating element disposed within the pre-heat chamber adjacent the at least a portion of the first liquid flow duct.
Clause 12. A personal vaporizer according to claim 11 wherein the at least one liquid preheat heating element comprises a coil heating element surrounding a circumference of the at least a portion of the first liquid flow duct.
Clause 13. A personal vaporizer according to claim 12 wherein the at least one liquid preheat heating element is configured to heat vaporizable liquid flowing through the first liquid flow duct to a liquid temperature in a range of 75-90 °F.
Clause 14. A personal vaporizer according to claim 12 wherein the at least one liquid preheat heating element is configured to heat vaporizable liquid flowing through the first liquid flow duct to a liquid temperature in a range of 78-82 °F.
Clause 15. A method of vaporizing a vaporizable liquid in a personal vaporizer having a liquid reservoir containing the vaporizable liquid, a vaporization chamber, and a vaporization heating element disposed within the vaporization chamber, the method comprising:
   drawing the vaporizable liquid from the liquid reservoir using an internal pumping mechanism;
   pre-heating, by a first heating element, the vaporizable liquid to a temperature within a predetermined temperature range;
   passing the vaporizable liquid through a nozzle into the vaporization chamber adjacent the vaporization heating element; and
   heating, by a second heating element, the vaporizable liquid above a vaporization temperature, thereby producing a vapor product.
Clause 16. A method according to claim 15 further comprising:
   mixing the vapor product with air drawn from outside the personal vaporizer to form an air-vapor mixture within the vaporization chamber; and
   exhausting the air-vapor mixture from the vaporization chamber.
Clause 17. A method according to claim 15 wherein the predetermined temperature range is 75-90 °F.
Clause 18. A method according to claim 15 wherein the predetermined temperature range is 78-82 °F.
Clause 19. A method according to claim 15 wherein the internal pumping mechanism is a piezoelectric pump,
Clause 20. A method according to claim 15 wherein the vaporizable liquid flows through the nozzle at a predetermined flow rate.

## Claims

1. A personal vaporizer comprising:
an annular main body defining a main body interior;
a vaporization chamber within the main body interior;
a vaporization heating element disposed within the vaporization chamber, the vaporization heating element having a vaporization heating surface;
an air flow passage from one or more air intake openings in the case wall to the vaporization chamber;
a vaporization mixture flow passage extending from the vaporization chamber to an exit port;
a liquid reservoir configured for storage of a vaporizable liquid and having a reservoir exit port;
a liquid transport arrangement for transporting vaporizable liquid from the liquid reservoir to the vaporization chamber, the liquid transport arrangement comprising
a first liquid flow duct extending from the reservoir exit port, and
a piezoelectric pump operatively connected to the first liquid flow duct for drawing the vaporizable liquid from the reservoir, through the liquid flow duct, and into the vaporization chamber through a nozzle positioned adjacent or in contact with the vaporization heating element so that liquid exiting the nozzle is heated by the vaporization heating surface.

2. A personal vaporizer according to claim 1 further comprising:
a pump controller in electrical communication with the piezoelectric pump and configured for controlling the piezoelectric pump so as to maintain a desired flow rate of liquid through the nozzle.

3. A personal vaporizer according to claim 2 wherein the desired flow rate is in a range from zero flow to a predetermined maximum flow rate.

4. A personal vaporizer according to claim 1 wherein the liquid transport arrangement further comprises:
a pre-heat chamber having a pre-heat chamber inlet in fluid communication with the first liquid flow duct and a pre-heat chamber outlet;
a second liquid flow duct in fluid communication with the pre-heat chamber outlet and the piezoelectric pump for transporting the vaporizable liquid therebetween; and
a liquid preheat heating element disposed within the pre-heat chamber.

5. A personal vaporizer according to claim 4 wherein the pre-heat chamber and the liquid preheat heating element are configured to heat the vaporizable liquid to a liquid temperature in a range of 75-90 °F.

6. A personal vaporizer according to claim 4 wherein the pre-heat chamber and the liquid preheat heating element are configured to heat the vaporizable liquid to a liquid temperature in a range of 78-82 °F.

7. A personal vaporizer according to claim 1 wherein the liquid transport arrangement further comprises:
a pre-heat chamber within which at least a portion of the first liquid flow duct is disposed; and
at least one liquid preheat heating element disposed within the pre-heat chamber adjacent the at least a portion of the first liquid flow duct.

8. A personal vaporizer according to claim 7 wherein the at least one liquid preheat heating element comprises a coil heating element surrounding a circumference of the at least a portion of the first liquid flow duct.

9. A personal vaporizer according to claim 8 wherein the at least one liquid preheat heating element is configured to heat vaporizable liquid flowing through the first liquid flow duct to a liquid temperature in a range of 75-90 °F.

10. A personal vaporizer according to claim 8 wherein the at least one liquid preheat heating element is configured to heat vaporizable liquid flowing through the first liquid flow duct to a liquid temperature in a range of 78-82 °F.

11. A method of vaporizing a vaporizable liquid in a personal vaporizer having a liquid reservoir containing the vaporizable liquid, a vaporization chamber, and a vaporization heating element disposed within the vaporization chamber, the method comprising:
drawing the vaporizable liquid from the liquid reservoir using an internal pumping mechanism;
pre-heating, by a first heating element, the vaporizable liquid to a temperature within a predetermined temperature range;
passing the vaporizable liquid through a nozzle into the vaporization chamber adjacent the vaporization heating element; and
heating, by a second heating element, the vaporizable liquid above a vaporization temperature, thereby producing a vapor product.

12. A method according to claim 11 further comprising:
mixing the vapor product with air drawn from outside the personal vaporizer to form an air-vapor mixture within the vaporization chamber; and
exhausting the air-vapor mixture from the vaporization chamber.

13. A method according to claim 11 wherein the predetermined temperature range is 75-90°F.

14. A method according to claim 11 wherein the predetermined temperature range is 78-82 °F.

15. A method according to claim 11 wherein the internal pumping mechanism is a piezoelectric pump.
